# EUROPEAN PATENT APPLICATION

(11) **EP 3 889 970 A1**
(43) Date of publication of application: **06.10.2021**
(21) Application number: 20168039.4
(22) Date of filing: 03.04.2020
(51) Int. Cl.: G16H 80/00, G16H 10/60, A61B 3/113, A61B 5/16, G06F 3/01

(54) **DIAGNOSIS SUPPORT SYSTEM**

(71) Applicant: Koninklijke Philips N.V., 5656 AG Eindhoven (NL)
(72) Inventor: TALGORN, Elise Claude Valentine, 5656 AE Eindhoven (NL); IN 'T GROEN, Robertus Leonardus Maria, 5656 AE Eindhoven (NL); HIWALE, Sujitkumar, 5656 AE Eindhoven (NL)
(74) Representative: Philips Intellectual Property & Standards

(57) **Abstract**

Some embodiments are directed to a diagnosis support system 100. The diagnosis support system 100 comprises a sensor subsystem 120 configured to detect a gaze of a user and to monitor a patient, a display subsystem 130 and a processor subsystem 110. The processor subsystem 110 is configured to track the gaze of the user, detect a patient parameter by monitoring the patient and determine an awareness level for the detected patient parameter based on the tracked gaze. The awareness level indicates the user's awareness level of the detected patient parameter. If the awareness level of the patient parameter is below a threshold awareness, the diagnosis support system 100 is configured to display information indicating the detected patient parameter.

## Description

### FIELD OF THE INVENTION

The presently disclosed subject matter relates to a diagnosis support system, particularly for detecting awareness of patient parameters, and a corresponding computer-implemented method. The presently disclosed subject matter further relates to a computer-readable medium.

### BACKGROUND OF THE INVENTION

In the first seconds of a consult, an experienced medical professional retrieves a large amount of information about a patient from inspection of their physical presentation, such as their gait, stature, posture, facial features and expression, hands and nails aspects, skin color, handshake, clothing, quality of voice, and smell. From these observations, the physician may create a mental list of the patient's characteristics, symptoms and potential problems. Also when initiating the dialogue with the patient (for example, during about the first fifteen second of a consult), the physician can gather additional information about the patient's mental and physical well-being from characteristics such as ease of speech, state of their mouth and their breathing pattern.

Physicians are now dealing with an ever-increasing workload, as well as shorter and shorter durations for consultations. However, it requires time to gain a full understanding of the patient's symptoms, concerns and related events. Missing important information due to time constraints may lead to misdiagnosis. Additionally, physicians may miss symptoms if they are displayed outside of the consultation, such as in a waiting room or the like. Hence, there is a clear benefit for a diagnosis support system that enables the physician to arrive at a correct diagnosis by complementing their observations with relevant data.

The concept of an augmented-reality (AR)-presented diagnosis support system is explored herein and may be applied with a head-mounted AR device or a standard mobile device, such as a tablet or a phone. The diagnosis support system may provide information about the patient lifestyle, a health condition, and physiological parameters when the physician does the initial diagnosis.

Published US patent application US 2018/0197624 A1 describes a wearable device which can present virtual content to the wearer for many applications in a healthcare setting. The wearable device may display patient medical records for a particular patient, and may be configured to use facial recognition to identify said patient. Additionally, the wearable device may be used to record an interaction between a health care provider and a patient, using initiation and termination events as triggers. In another embodiment, the wearable device may display relevant portions of a patient's medical record, or patient physiological data, to the health care provider.

A problem of the prior art is that the wearable device does not actively observe physiological patient parameters, nor does it take into account information that the physician has already observed.

Providing every observation made by the system to the physician would overload the physician with information, much of which the physician has either already noticed or is not relevant. There is therefore a need to provide the physician with observational information and/or physiological information of a patient whilst determining what observations have already been observed by the physician, such that displaying unnecessary data and therefore overloading the physician may be avoided.

### SUMMARY OF THE INVENTION

It would be advantageous to have a diagnostic support system which identifies a patient parameter (or a plurality of patient parameters) to support a physician's diagnostic process. A diagnosis support system and computer-implemented method are set out herein and are claimed that aim to address these and other concerns.

Existing medical assistant systems and devices do not directly identify patient parameters, and are instead limited to facial recognition, to identify a patient, and/or speech recognition, to determine based on keywords when an interaction between a patient and physician is to be recorded. It would therefore be advantageous to have a diagnosis support system configured to identify a patient parameter by observing a patient, and to determine what information relating to the identified patient parameter should be observed, based on a user's detected awareness to the patient parameter. A technical problem addressed by the presently disclosed subject matter would be to improve a user's access to diagnostic information.

The presently disclosed subject matter includes a diagnosis support system, a computer-implemented method and a computer-readable medium. The diagnosis support system may be configured to identify a patient parameter using a sensor system and observe the patient, and to determine an awareness of a user of the diagnosis support system with regard to the identified patient parameter. For example, the diagnosis support system may identify a swelling on a patient's left arm, and may determine whether the user is aware of the swelling. The diagnosis support system may determine the awareness of the user by tracking a gaze of the user, in order to determine whether the user has observed the patient parameter (e.g. the diagnosis support system may track the gaze of the user to determine if the user has looked at the patient's left arm and has therefore noticed the swelling).

Embodiments help to improve an information yield obtained from an interaction between a patient and a user (e.g. a physician or a health care provider), whilst avoiding overloading the user with information. The diagnosis support system is configured to identify and recognize a patient parameter as well as to determine whether information corresponding to the patient parameter is to be provided to the user (e.g., physician or health care provider), using information such as a detected gaze of the user.

In an embodiment, the diagnosis support system comprises a sensor subsystem, a display subsystem and a processor subsystem. The sensor subsystem is configured to detect a gaze of a user of the diagnosis support system and to monitor a patient. The processor subsystem is configured to track, via the sensor subsystem, the gaze of the user, detect a patient parameter by monitoring the patient using the sensor subsystem, and determine an awareness level for the detected patient parameter based on the tracked gaze. The awareness level indicates the awareness level of the user with regard to the patient parameter, e.g. how aware the user is of the detected patient parameter. If the awareness level of the patient parameter is below a threshold awareness, the processor subsystem is configured to display, via the display subsystem, information indicating the detected patient parameter.

By tracking the gaze of the user, the diagnosis support system may determine if the user of the system has observed an area of the patient corresponding to a detected patient parameter. Based on this determination, the system may or may not display information indicating the detected patient parameter to the user. This may reduce the amount of unnecessary (e.g. redundant) information provided to the user.

By monitoring the patient using the sensor subsystem, information that may otherwise be missed or that may require further attention may be observed and, optionally, recorded. Thus, the user is assisted in noticing irregularities, characteristics, data and contextual information which may contribute to his or her diagnosis.

Aspects of the presently disclosed subject matter include a computer implemented method.

Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

The system of claim 1 may exist as a single device or as a plurality of devices which are communicatively coupled, such as in a network. One or more components of the system of claim 1 may be provided within a single device whilst other components are provided in one or more further devices communicatively coupled to the single device. For example, the system of claim 1 may be provided in a wearable device configured to be worn by the user, such as a head-mounted display or a pair of smart glasses. In another example, the system of claim 1 is provided in a mobile device, such as a tablet or mobile phone.

In an embodiment, the computer program comprises computer program code adapted to perform all or part of the steps of an embodiment of the method when the computer program is run on a computer. Preferably, the computer program is embodied on a computer readable medium.

Another aspect of the presently disclosed subject matter provides a method of making the computer program available for downloading. This aspect is used when the computer program is uploaded into, e.g., Apple's App Store, Google's Play Store, or Microsoft's Windows Store, and when the computer program is available for downloading from such a store.

### BRIEF DESCRIPTION OF THE DRAWINGS

Further details, aspects, and embodiments will be described, by way of example, with reference to the drawings. Elements in the figures are illustrated for simplicity and clarity and have not necessarily been drawn to scale. In the Figures, elements which correspond to elements already described may have the same reference numerals. In the drawings,
Figure 1 schematically shows an example of an embodiment of a diagnosis support system,
Figure 2 schematically shows an example of an embodiment of a diagnosis support system comprising a mobile device,
Figure 3 schematically shows an example of an embodiment of a diagnosis support system comprising an augmented-reality device,
Figure 4 schematically shows an example of an embodiment of a diagnosis support system comprising multiple components,
Figure 5 schematically shows an example of an embodiment of a diagnosis support method,
Figure 6 schematically shows an example of an embodiment of an awareness determining method,
Figure 7 schematically shows an example of an embodiment of a diagnosis support method,
Figure 8 schematically shows an example of an embodiment of a diagnosis support method,
Figure 9 schematically shows a computer readable medium having a writable part comprising a computer program according to an embodiment, and
Figure 10 schematically shows a representation of a processor system according to an embodiment.

### List of Reference Numerals:

- 10: a patient
- 20: a user
- 100: a diagnosis support system
- 110: a processor subsystem
- 120: a sensor subsystem
- 121: a camera
- 123: a sound sensor
- 125: a patient monitoring sensor
- 130: a display subsystem
- 140: a data interface
- 150: a memory
- 151: a patient database
- 153: a medical database
- 200: a mobile device
- 210: a live view of a patient
- 220: a front-facing camera
- 230: a screen of a mobile device
- 240: information on a patient parameter
- 250: information from a patient record
- 300: a head-mounted display
- 310a,b: at least one sensor for observing a patient
- 320: a sensor for tracking an eye gaze of a user
- 330: a display
- 400: an additional sensor
- 1000: a computer readable medium
- 1010: a writable part
- 1020: a computer program
- 1110: integrated circuit(s)
- 1120: a processing unit
- 1122: a memory
- 1124: a dedicated integrated circuit
- 1126: a communication element
- 1130: an interconnect
- 1140: a processor system
- 1100: a device

### DETAILED DESCRIPTION OF EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them.

Further, the presently disclosed subject matter is not limited to the embodiments, as features described herein or recited in mutually different dependent claims may be combined.

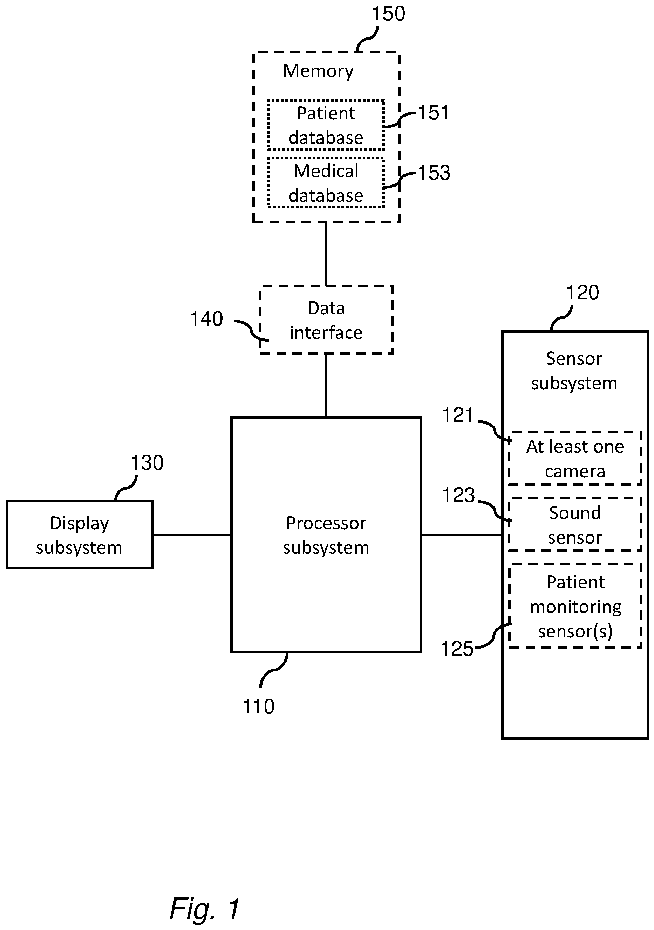

**Figure 1** schematically shows an example of an embodiment of a diagnosis support system 100. Diagnosis support system 100 comprises a processor subsystem 110, a sensor subsystem 120 and a display subsystem 130.

In an embodiment, sensor subsystem 120 may comprise at least one sensor component, for example at least one of a camera 121, a sound sensor 123 and a temperature sensor 125. Although not shown in Fig. 1, the sensor subsystem 120 may comprise other sensors, such as a patient monitoring sensor, a biometric sensor, a pressure sensor, a distance sensor, and the like. The sensor subsystem 120 may comprise a plurality of cameras, sound sensors and/or patient monitoring sensors. In an embodiment, the sensor subsystem 120 may comprise at least one sensor disposed outside of the consultation room, such as in a waiting area or the like. Patient parameters observed outside of the consultation room, e.g. when the user (e.g. health care provider, doctor, nurse etc.) is not present, may then be indicated to the user.

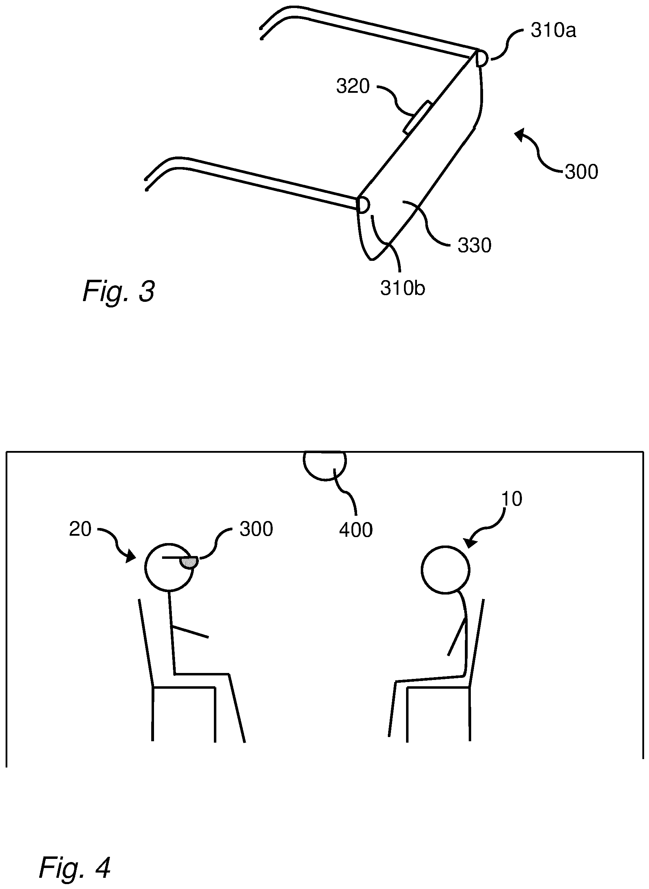

In an embodiment, sensor subsystem 120 may comprise a camera configured to track a gaze of a user and monitor a patient. For example, the camera may be multidirectional. For example, the camera may be disposed in room, such as on a ceiling or wall, and arranged to monitor the entirety of the room. One such example will be described in detail with reference to Figure 4.

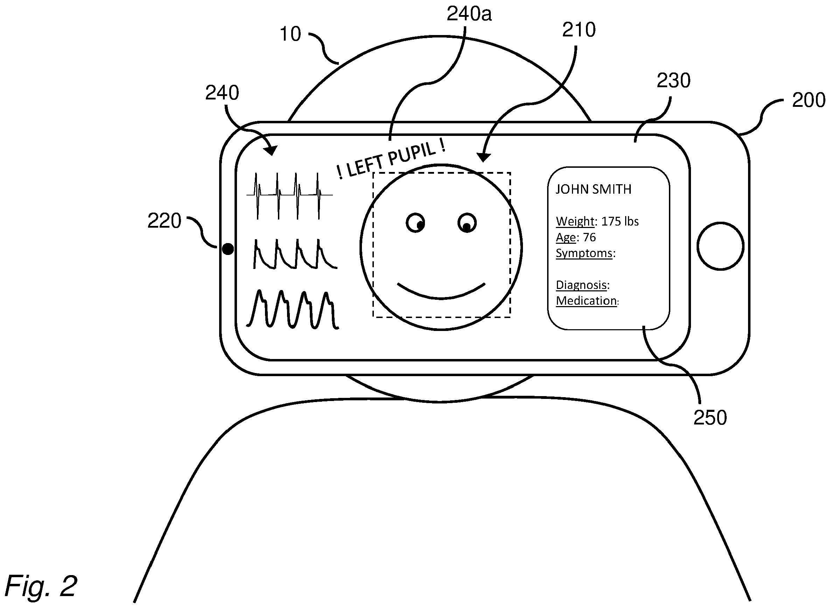

In an embodiment, sensor subsystem 120 may comprise at least two cameras 121. For example, a first camera of the sensor subsystem 120 may be configured to track a gaze of a user of the diagnosis support system 100, whilst a second camera of the sensor subsystem 120 may be configured to monitor a patient. For example, the first camera and the second camera may face different directions. The first camera may be arranged to face the user whilst the second camera may be arranged to face a patient. In an embodiment, one or both of the first and second cameras may be disposed on a single device, such as on a head-mounted display (HMD) (discussed with regard to Figure 3) or in a mobile device (discussed with regard to Figure 2).

In an embodiment, sensor subsystem 120 may comprise a plurality of cameras 121 affixed in a plurality of locations. For example, a room such as a consultation room may be equipped with at least one camera, arranged to observe the room. A further camera may be disposed on a wearable device worn by either a patient or the user (e.g. a doctor or a health care provider). An additional camera, arranged to focus on a particular area of the room, such as a bed on which a patient may be examined, may be further disposed in the room. The additional camera may be independently controlled, or may have a different parameter, such as higher resolution or a different zoom level, compared to the other camera(s). In an embodiment, sensor subsystem 120 may comprise a camera in an external location, such as in a waiting room or reception area.

A camera 121 of the sensor subsystem 120 may comprise an infrared sensor configured to track a gaze of a user by detecting a reflection of light, such as infrared or near-infrared light, from the user's cornea, for example by using pupil center corneal reflection. The gaze tracking may also be referred to as focus tracking, eye tracking, attention tracking or similar. Tracking a gaze may comprise measuring a rotation of a user's eye. In an embodiment, gaze tracking comprises determining a head direction and determining an eye-in-head direction (e.g. angle of eye rotation with respect to the user's head).

A camera 121 of the sensor subsystem 120 may comprise an optical camera configured to monitor a patient, and to detect a patient parameter, such as a gesture made by the patient, a tic or twitch, a facial expression (such as a grimace), a facial characteristic (such as complexion, skin tone, presence of freckles, irregular blinking or winking, or asymmetrical facial expressions), a height, a weight, a clothing characteristic (such as an amount of clothing, whether clothing worn by the patient is warm-weather clothing or cold-weather clothing, a logo on the clothing), a hair characteristic (such as hair thinning, balding, hair shine, hair length, presence or absence of facial hair, etc.), a fingernail characteristic (such as presence of ridges or holes, nail bed color, presence of discoloration or ingrown nail, etc.), a gait, and a physical anomaly such as a rash or swelling on the patient's body. Further examples of patient parameters are envisaged.

In an embodiment, a camera 121 of the sensor subsystem 120 may be configured to recognize a patient or a user by using facial recognition.

In an embodiment, sensor subsystem 120 may comprise a sound sensor 123, such as a microphone. The sound sensor 123 may be configured to detect sound emitted by the user and/or the patient. The sound sensor 123 may comprise one or more microphones. For example, a microphone may be worn by the user and/or the patient. For example, a microphone may be integrated in a mobile device or a wearable device such as a head-mounted display. For example, one or more microphones may be installed in one or more locations of a room such as a consulting room.

In an embodiment, a sound sensor 123 of the sensor subsystem 120 may be configured to detect a speech of a user (e.g. a doctor or a health care provider) and/or a patient. The sound sensor 123 may be configured to use speech recognition and, for example, natural language processing (NLP) or keyword determination to infer contextual information.

In an embodiment, a sound sensor 123 of the sensor subsystem 120 may be configured to detect an audible patient parameter from the patient, such as breathing sounds, wheezing, coughing, heart sounds, stomach and/or bowel sounds. The audible patient parameter may comprise, for example, speech characteristics such as slurring, vocal tone, vocal pitch, vocal tics, repetitions, hesitations (e.g. "umm", "hmm", etc.), stammers, speech impediments, vocal volume and vocal tremor.

In an embodiment, sensor subsystem 120 may comprise one or more patient monitoring sensors, configured to detect a patient parameter. The patient parameter may be, for example, a heart rate, a blood pressure, a height, a weight, a breathing rate, an oxygen saturation level, a body temperature, a tendon reflex, a circulation and the like.

The sensor subsystem 120 may comprise any or all of the above-described sensors, and may comprise additional sensors. The sensor subsystem 120 may comprise one of the above-described sensors or any combination of sensors. The sensor(s) of the sensor subsystem 120 may be disposed on a single device, or may take the form of separate devices.

The diagnosis support system 100 may comprise a display subsystem 130. The display subsystem 130 may comprise a screen for displaying text and/or image data. The screen may be communicatively coupled to a user input/output system. The screen may be a touchscreen.

In an embodiment, display subsystem 130 may be configured to display text and/or images using augmented reality (AR). That is, the display subsystem 130 may be arranged to overlay text and/or image data to be displayed onto a live image, for example received by a camera or other sensor connectively coupled to the system 100, for example sensor subsystem 130. The overlay may be a virtual overlay.

For example, the display subsystem 130 may comprise a screen of a head-mounted display (HMD), such as in the form of glasses or a visor. The HMD may be at least partially transparent or translucent, allowing a wearer of the HMD to see through the HMD. The view of the wearer may be tinted or may be unaffected by the presence of the HMD The HMD may display text and/or image data to the wearer as a virtual overlay. In other words, text and/or image data may be displayed to the wearer as overlaid on their view of their environment, for example if the wearer is looking at an object, information about the object may be displayed next to or on top of (e.g. overlaid on) the obj ect.

In an embodiment, the display subsystem 130 may comprise a screen of a mobile device, such as a tablet or a mobile phone. Text and/or image data may be displayed on the screen of the mobile device. For example, a user may direct a camera of the mobile device toward a patient and be presented with a live or still (e.g. captured) image or video of the patient. Additional information, such as text and/or image data, may be displayed overlaid on at least one relevant area of the patient. In this example, the user would then see the patient and the additional information simultaneously in the screen of the mobile device.

The diagnosis support system 100 may comprise a processor subsystem 110. The processor subsystem 110 may be electrically coupled, either wirelessly or wired, to the display subsystem 130 and the sensor subsystem 120. The processor subsystem 110 may be electrically coupled to a data interface 140.

The processor subsystem 110 may comprise at least one processor, also referred to as at least one processor circuit.

In an embodiment, the processor subsystem 110 may be configured to control the sensor subsystem 120 to track a gaze of a user. For example, the processor subsystem 110 may transmit a signal to the sensor subsystem 120 triggering a gaze tracking operation.

The processor subsystem 110 may be configured to control the sensor subsystem 120 to monitor a patient and in doing so, to detect a patient parameter.

In an embodiment, the processor subsystem 110 may be configured to control the sensor subsystem 120 such that the sensor subsystem 120 simultaneously tracks the gaze of the user and monitors the patient to detect the patient parameter. In an embodiment, multiple patient parameters are detected.

The processor subsystem 110 may be configured to determine an awareness level of the user to the detected patient parameter. For example, if the user's gaze is tracked to a particular region of the patient's body, such as a left hand, and a patient parameter such as a rash is detected on the patient's left hand, the processor subsystem 110 may determine that the user is aware of the detected patient parameter (e.g. the user has observed the detected patient parameter). The determination may be at least partially based on an amount of time for which the gaze of the user is detected on the relevant area or region of the patient, such as a first threshold time. If the gaze of the user is not detected at the relevant region or area of the patient, or if the gaze of the user is not detected at the relevant region or area for at least the first threshold time, the processor subsystem 110 may determine that the user is not aware of the patient parameter, e.g. the user has not seen the patient parameter. The use of a threshold time may prevent an accidental or fleeting glance at an area of the patient from being interpreted as the user being aware (or having noticed) a patient parameter.

In an embodiment, the threshold time may comprise a learned behavior. For example, if the diagnosis support system 100 determines that the user is unaware of a patient parameter because their gaze was not detected for the threshold time but the user is actually aware of said patient parameter, the user may indicate to the system that said patient parameter was noticed. Based on this feedback, the diagnosis support system 100 may adapt or adjust (for example, reduce) the threshold time for subsequent use. For example, the diagnosis support system 100 may use a neural network, machine learning or a support vector machine in order to determine the threshold time based on the acquired feedback. In an embodiment, the processor subsystem 110 may be configured to collect gaze time information indicating a time required for the user to notice the patient parameter. For example, the gaze time information may be the time during which the gaze of the user is detected on the corresponding area or region of the patient's body and then indicating an awareness explicitly, or providing an implicit indication of their awareness. The indication of their awareness may comprise a comment detected by a sound sensor, an entry in a patient record, or feedback to the system as discussed as above, for example. The processor subsystem 110 may be further configured to adapt the threshold time based on the collected gaze time information. For example, if the user indicates awareness of a patient parameter after only 5 seconds whereas the threshold time is initially set to 10 seconds, the threshold time may be reduced, for example to 5 seconds or by a smaller increment. The reduction may be based on previously received feedback.

In an embodiment, if the processor subsystem 110 (via the sensor subsystem 120) detects a recurring gaze of the user on the relevant region or area of the patient corresponding to the detected patient parameter, and/or a gaze on the relevant region or area of the patient lasting longer than a second threshold time (the second threshold time being longer than the first threshold time), the processor subsystem 110 may determine that the user is uncertain about the patient parameter, and may accordingly determine the awareness level to be uncertain.

In an embodiment, if the processor subsystem 110 determines the user's awareness of a detected patient parameter to be below a threshold awareness, for example if the processor subsystem 110 determines that a user is unaware or uncertain of the detected patient parameter, the processor subsystem 110 may control the display subsystem 130 to display information indicating the detected patient parameter. Referring back to the example above, if the processor subsystem 110 determines that the user is unaware of a rash on a patient's left hand (e.g. if the user's gaze has not been detected on the patient's left hand), the processor subsystem 110 may control the display subsystem 130 to display an alert, such as "RASH ON LEFT HAND", or a visual indicator, such as an arrow pointing to the left hand.

In an embodiment, if the processor subsystem 110 determines that the user is aware of the detected patient parameter, e.g. if the user's awareness of the detected patient parameter is above a threshold awareness, then no information about the detected patient parameter may be displayed via the display subsystem 130. In this way, information overload may be reduced or prevented - if the user has noticed the patient parameter, there is no need to draw the user's attention to the patient parameter again.

In an embodiment, further information such as sound information (e.g. speech recognition) and other contextual information may be used in the determination of a user's awareness level. For example, if a user mentions a detected patient parameter, the processor subsystem 110 may determine that the user is aware of the detected patient parameter, regardless of whether the user's gaze has been detected on the relevant region or part of the patient. Contextual information may take the form of diagnostic tests being ordered or medications being prescribed. The processor subsystem 110 may receive information regarding diagnostic tests being ordered and/or medications being prescribed, and this information may contribute to the determination of the user's awareness level of the patient parameter. For example, if the user orders a test corresponding to the patient parameter, such as an x-ray of a swollen ankle, the diagnosis support system 100 may determine that the user (e.g. a doctor or health care provider) has noticed a patient parameter indicating a swollen ankle.

The diagnosis support system 100 may further comprise a data interface 140. The data interface 140 may comprise a connector, e.g., a wired connector, e.g., an Ethernet connector, an optical connector, etc., or a wireless connector, e.g., an antenna, e.g., a Wi-Fi, 4G or 5G antenna. The data interface 140 may provide access to a memory 150.

The memory 150 may comprise at least one database, such as a patient database 151 and/or a medical database 153. The patient database 151 may comprise patient records for at least one patient. The medical database 153 may comprise information, such as symptoms, diagnostic information and treatments, on at least one ailment or disease.

In an embodiment, the diagnosis support system 100 may comprise a memory 150. The memory 110 may be implemented as an electronic memory, for example a flash memory, or magnetic memory, say hard disk or the like, or optical memory, e.g., a DVD. The memory 110 may comprise multiple discrete memories together making up the memory 110. The memory110 may comprise a temporary memory, say a RAM. In the case of a temporary memory 110, memory 110 may be associated with a retrieving device to obtain data before use and to store the data in the storage, say by obtaining them over an optional network connection (not shown).

In an embodiment, the memory 150 may comprise a local memory and/or an external (e.g. remote) memory. For example, the patient database 151 may be stored in a local memory, which may ensure patient privacy. The medical database 153 may be stored externally and merely accessed by the diagnosis support system 100. In another example, the patient database 151 may be stored externally, such as in a central (e.g. government) database. The patient database 151 and the medical database 153 may be stored in the same storage location, or in different storage locations.

The various subsystems of the diagnosis support system 100 may be disposed within a single device, or may communicate with each other over a computer network. The computer network may be an internet, an intranet, a LAN, a WLAN, etc. The computer network may be the Internet. The computer network may be wholly or partly wired, and/or wholly or partly wireless. For example, the computer network may comprise Ethernet connections. For example, the computer network may comprise wireless connections, such as Wi-Fi, ZigBee, and the like. The subsystems may comprise a connection interface which is arranged to communicate with other subsystems of system 100 as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna. The computer network may comprise additional elements, e.g., a router, a hub, etc.

**Figure 2** schematically shows an example of an embodiment of a diagnosis support system comprising a mobile device 200.

Figure 2 illustrates a view from the perspective of a user examining a patient 10 using a mobile device of the diagnosis support system. For example, the diagnosis support system 100 may be completely implemented within the mobile device 200, or the mobile device 200 may be a component of the diagnosis support system 100. In the example of Figure 2, the user may direct a rear-facing camera (not shown) of the mobile device 200 towards the patient 10. A live view 210 of the patient 10, such as an image or video, may be displayed on a screen 230 of the mobile device 200.

In an embodiment, information on at least one detected patient parameter 240 may be displayed on the screen 230. In the example shown in Figure 2, a heartrate, an oxygen saturation and a blood pressure graph are shown as information 240.

In an embodiment, the mobile device 200 may further comprise a front-facing camera 220. The front-facing camera 220 may be comprised in sensor subsystem 120. The front-facing camera 220 may be configured to track a gaze of the user, whilst the rear-facing camera may be configured to monitor the patient 10 and detect a patient parameter. The front-facing camera 220 may comprise an infrared scanner.

If the user's gaze is not detected on an area of the patient corresponding to the detected patient parameter, an alarm, such as an alert 240a, may be displayed on the screen 230. In this example, the patient presents with muscle weakness in his left eye, resulting in an asymmetry as shown. If the diagnosis support system 100 (implemented in this example as mobile device 200) does not detect the user's gaze on the patient's left eye, the system 100 may determine that the user is unaware of the patient parameter of the eye asymmetry. An alarm or alert 240a may then be shown in order to direct the user's attention to the detected patient parameter.

In an embodiment, the diagnosis support system 100 may be configured to track the gaze time required by the user to notice (e.g. become aware) of the patient parameter. For example, the gaze time may be tracked according to the type of patient parameter, the area of the body on which the patient parameter presents, or the like. In an embodiment, gaze time tracking information from a plurality of users, such as users of a plurality of diagnosis support systems 100 or the same diagnosis support system 100, may be combined in order to determine an average gaze time. The average gaze time may, for example, be based on the type of patient parameter, the area of the body on which the patient parameter presents, or a more general gaze time. The average gaze time may then be used to refine or adjust the threshold time used to determine whether a user is aware of a patient parameter.

In an embodiment, at least one sensor of the sensor subsystem 120 is external to the mobile device 200, and may be electrically connected to the mobile device 200, for example via a network (such as a wireless network, the Internet, a local area network or the like) or via wireless communication (such as Bluetooth, Zigbee, NFC or the like).

In an embodiment, the diagnosis support system 100 may comprise at least one external sensor disposed in an area outside of the main consultation room. For example, the at least one external sensor may be disposed in a waiting room, an entrance area, a triage area or the like. If the at least one external sensor detects a patient parameter, the diagnosis support system 100 may then pay particular attention to said patient parameter during the consultation. If the user remains unaware of said patient parameter, the diagnosis support system 100 may display information indicating said patient parameter to the user. The at least one sensor disposed within the consultation room, such as on the mobile device 200, may be used to track the gaze of the user and to determine whether the user becomes aware of the detected patient parameter.

In an embodiment, the diagnosis support system 100 may be configured to retrieve information from a patient record, such as a patient record stored in the patient database 151. For example, the patient 10 may be identified by information in an appointment calendar, or the patient 10 may be identified using facial recognition. Information 250 from the patient record, such as a medical history and other details, may be displayed on the screen 230.

In an embodiment, the diagnosis support system 100 may be configured to save the patient parameter detected during a consultation to the patient record. For example, the diagnosis support system 100 may store details of the patient's left eye muscle weakness in the patient record. The details may include a photograph or a visual and/or audio capture of the consultation. If multiple patient parameters are detected, some or all of them may be saved or stored in the patient record.

The mobile device 200 may comprise some or all of the components of the diagnosis support system 100. In an example, the mobile device 200 may be coupled to additional sensors.

**Figure 3** schematically shows an example of an embodiment of a diagnosis support system comprising an augmented-reality device.

Figure 3 shows an augmented reality device, such as a head-mounted display (HMD) device 300. The HMD device 300 may be configured to be worn by the user 20. The HMD device 300 may comprise at least one sensor 310. For example, sensor 310a may be a camera, and sensor 310b may be a microphone. In another example, both sensors 310a and 310b may be cameras, or one or more of the sensors 310a and 310b may comprise a combination of a camera and a microphone. The HMD device 300 may be equipped with one of sensors 310a and 310b or both sensors 310a and 310b, or with additional sensors. At least one of sensor 310a and 310b may be configured to monitor the patient 10 and to detect at least one patient parameter.

In an embodiment, at least one of sensors 310a and 310b may be configured to perform facial recognition in order to identify the patient 10. For example, the HMD device 300 may be configured to identify the patient 10 through facial recognition and access the associated patient record from memory, such as memory 150. Additionally or alternatively, the patient 10 may be identified via scheduling information.

In an embodiment, the HMD device 300 may comprise a further sensor 320 configured to track an eye gaze of the user 20. For example, the sensor 320 may be arranged to face the wearer of the HMD device 300, whilst the at least one sensor 310 may be arranged to observe and/or capture the user's field of view.

In an embodiment, the HMD device 300 may comprise a display screen 330. The display screen 330 may be transparent or translucent, such that the user 20 is able to see the patient 10 through the display screen 330. The display screen 330 may be configured to display information, such as information 240 and/or information 250, as text and/or image data overlaid on the view of the user 20. For example, if the user 20, wearing the HMD device 300, is looking at a patient, information may be displayed in a relevant area of the user's view, such that it appears overlaid on the user's view of the patient.

In an embodiment, the display screen 330 of the HMD device 300 is at least substantially opaque, such that the user 20 is not able to clearly see the patient through the material of the display screen 330. The at least one sensor 310 may comprise a camera sensor, such as a camera sensor 310a, configured to capture a live video image of the patient 10 towards which the user 20 is looking. The live-capture video image of the patient 10 may be displayed, to the user 20, on the display screen 330. Information, such as information 240 and/or information 250, may be displayed as overlaid text and/or image data on the display screen 330.

In an embodiment, at least one of the sensors 310a and 310b may comprise a sound recording component, such as a microphone. The microphone may be configured to record an audible patient parameter and/or the discussion between the user 20 and the patient 10.

In an embodiment, information detected from at least one of sensors 310a, 310b and 320 may be stored in a patient record in memory, such as memory 150.

The HMD device 300 may comprise some or all of the components of the diagnosis support system 100. In an example, the HMD device 300 may be coupled to additional sensors.

**Figure 4** schematically shows an example of an embodiment of a diagnosis support system comprising multiple components.

Figure 4 shows an example of HMD device 300 in use during a consultation between a user 20 (such as a health care provider or doctor) and a patient 10. As shown in Figure 4, the user 20 may be wearing the HMD device 300.

The diagnosis support system 100 may comprise at least one additional sensor 400, in addition to or as an alternative to the HMD device 300. The additional sensor 400 may comprise at least one of a camera and a microphone. In an embodiment, the additional sensor 400 may be positioned such as to observe both the patient 10 and the user 20. For example, the additional sensor 400 may be configured to track the eye gaze of the user 20 as well as monitor the patient 10 and detect at least one patient parameter. In another example, the diagnosis support system 100 may comprise a plurality of sensors disposed in various positions around the room, such as microphones near an examining area and/or near the user's main workstation, and the like.

In an embodiment, the diagnosis support system 100 may comprise at least one external sensor disposed in an area outside of the main consultation room. For example, the at least one external sensor may be disposed in a waiting room, an entrance area, a triage area or the like. If the at least one external sensor detects a patient parameter, the diagnosis support system 100 may then pay particular attention to said patient parameter during the consultation. If the user remains unaware of said patient parameter, the diagnosis support system 100 may display information indicating said patient parameter to the user. The at least one sensor disposed within the consultation room, such as on the HMD device 300, may be used to track the gaze of the user and to determine whether the user becomes aware of the detected patient parameter.

In an embodiment, the HMD device 300 may be used as described with reference to Figure 3, and the at least one additional sensor 400 may be used in conjunction to the HMD device 300. The at least one additional sensor 400 may be electronically coupled to the HMD device 300, for example via a direct wireless connection or via an intermediate device or hub, such as a server.

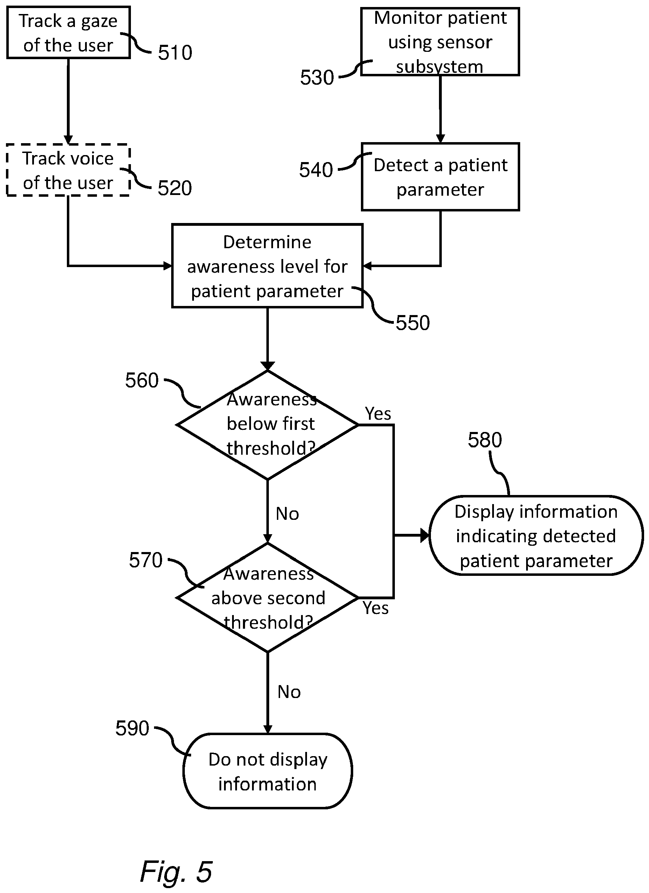

**Figure 5** schematically shows an example of an embodiment of a diagnosis support method.

In operation 510, the diagnosis support system 100 may be configured to track a gaze of the user 20. For example, at least one sensor of the sensor subsystem 120 may be arranged to track the eye gaze of the user 20. In optional operation 520, the diagnosis support system 100 may be configured to track a voice of the user 20. For example, at least one sensor of the sensor subsystem 120 may be arranged to track the user's voice, such as by performing speech recognition and/or by using keyword recognition. Operations 510 and 520 may be performed simultaneously. In an embodiment, contextual information such as information on diagnostic tests being ordered by the user 20 may also be tracked.

In operation 530, the diagnosis support system 100 may be configured to monitor (e.g. observe) a patient 10, for example via the sensor subsystem 120. The diagnosis support system 100, e.g. the sensor subsystem 120, may detect a patient parameter in operation 540. In an embodiment, a plurality of patient parameters may be detected by the sensor subsystem 120 in operation 540. Operations 510, 520, 530 and 540 may occur substantially simultaneously or simultaneously.

In operation 550, the diagnosis support system 100, via the processor subsystem 110, may determine an awareness level for the detected patient parameter. The tracked gaze of the user and, if available, the tracked voice of the user, may be used to determine if the user has noticed the detected patient parameter. For example, by tracking the eye gaze of the user, the diagnosis support system 100 may determine if the user has looked at an area or region of the patient's body corresponding to the detected patient parameter. As a further example, by tracking the voice of the user, the diagnosis support system 100 may determine if the user has noticed the detected patient parameter based on whether the detected patient parameter or the corresponding area or region of the patient's body has been mentioned aloud.

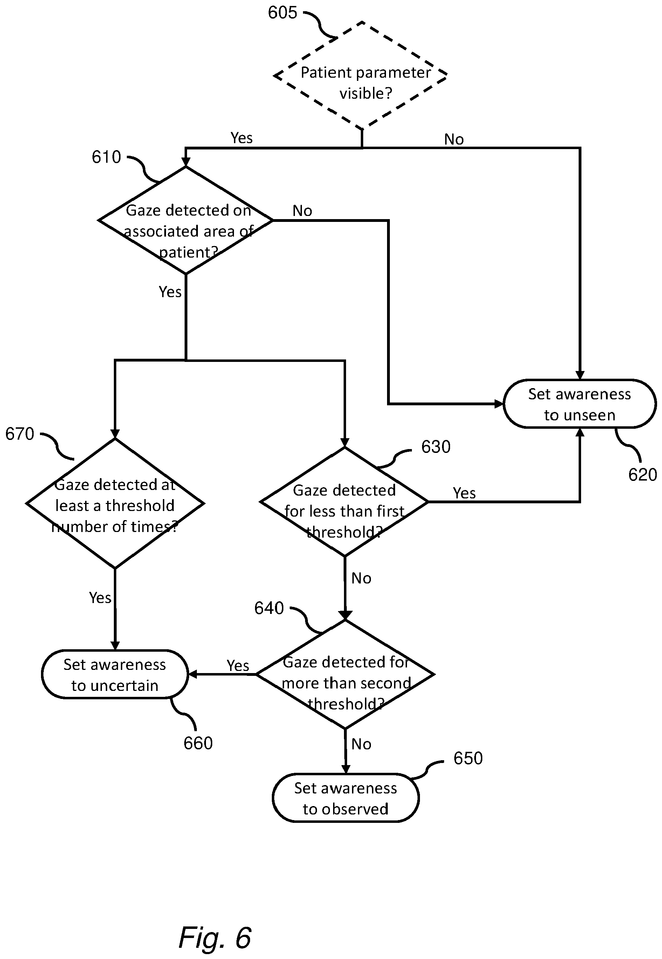

The determination of the awareness level of the user for the detected patient parameter may be based on at least one threshold. An example of a process of determining the awareness level will be described with reference to Figure 6. Returning to Figure 5, in operation 560, the diagnosis support system 100 may determine whether the user's awareness of the detected patient parameter is below a first threshold. If the user's awareness of the detected patient parameter is below the first threshold, for example if the user is deemed not to have noticed the detected patient parameter, the diagnosis support system 100 may be configured to display, via the display subsystem 130, information indicating the detected patient parameter, as shown in operation 580.

If the user's awareness is not below the first threshold (e.g., the user's awareness is at or above the first threshold), the diagnosis support system 100 may determine whether the user's awareness is above a second threshold, in operation 570. The second threshold is higher than the first threshold. For example, if the user's eye gaze is detected on the region or area of the patient's body corresponding to the detected patient parameter for a long time, the diagnosis support system 100 may determine that the user is uncertain about the detected patient parameter and that further information or attention may be necessary. If the user's awareness level is determined to not exceed the second threshold, the diagnosis support system 100 may be configured not to display information indicating the detected patient parameter, as shown in operation 590. Neglecting to display information that the user is sufficiently aware of may be helpful in preventing or reducing information overload. If the user's awareness level is above the second threshold, for example if the diagnosis support system 100 determines that the user is uncertain about the detected patient parameter, then the diagnosis support system 100 may be configured to display information indicating the detected patient parameter, via the display subsystem 130, as shown in operation 580.

In an embodiment, if the user's awareness of the detected patient parameter is determined to be above the second threshold, for example indicating that the user is uncertain about the detected patient parameter, the information indicating the detected patient parameter may be different than the information that would be displayed if the diagnosis support system determined the user's awareness to be lower than the first threshold. For example, if the user's eye gaze frequently returns to the region or area of the patient's body corresponding to the detected patient parameter, and/or if the user's gaze is tracked to said region or area for at least some threshold amount of time, the system 100 may determine that the user is uncertain about the patient parameter. In this case, the information displayed in operation 580 may relate to further tests or examinations that may be performed to obtain more information, a patient history corresponding to said patient parameter, information on possible ailments or diseases, or the like.

In an embodiment, the diagnosis support system 100 may be configured to track the gaze time required by the user to notice (e.g. become aware) of the patient parameter. For example, the gaze time may be tracked according to the type of patient parameter, the area of the body on which the patient parameter presents, or the like. In an embodiment, gaze time tracking information from a plurality of users, such as users of a plurality of diagnosis support systems 100 or the same diagnosis support system 100, may be combined in order to determine an average gaze time. The average gaze time may, for example, be based on the type of patient parameter, the area of the body on which the patient parameter presents, or a more general gaze time. The average gaze time may then be used to refine or adjust the first threshold time used to determine whether a user is aware of a patient parameter.

In an embodiment, the first and/or second threshold time may comprise a learned behavior. For example, if the diagnosis support system 100 determines that the user is unaware of a patient parameter because their gaze was not detected for the first threshold time but the user is actually aware of said patient parameter, the user may indicate to the system that said patient parameter was noticed. Based on this feedback, the diagnosis support system 100 may adapt or adjust (for example, reduce) the threshold time for subsequent use. For example, the diagnosis support system 100 may use a neural network, machine learning or a support vector machine in order to determine the threshold time based on the acquired feedback. In an embodiment, the processor subsystem 110 may be configured to collect gaze time information indicating a time required for the user to notice the patient parameter. For example, the gaze time information may be the time during which the gaze of the user is detected on the corresponding area or region of the patient's body and then indicating an awareness explicitly, or providing an implicit indication of their awareness. The indication of their awareness may comprise a comment detected by a sound sensor, an entry in a patient record, or feedback to the system as discussed as above, for example. The processor subsystem 110 may be further configured to adapt the first threshold time based on the collected gaze time information. For example, if the user indicates awareness of a patient parameter after only 5 seconds whereas the first threshold time is initially set to 10 seconds, the first threshold time may be reduced, for example to 5 seconds or by a smaller increment. The reduction may be based on previously received feedback. Similarly, the diagnosis support system 100 may be configured to determine a gaze time corresponding to an uncertainty, in an analogous manner. The second threshold time may thus be similarly adjusted based on feedback from the user and/or from collected gaze information.

**Figure 6** schematically shows an example of an embodiment of an awareness determining method.

The method of Figure 6 refers to the use of eye gaze tracking in order to determine the awareness of a user for the detected patient parameter. However, it is envisaged that the speech of the user and/or the patient may optionally contribute to the determination of the awareness level. The basic method, using the eye gaze tracking, will be discussed first, followed by additional information regarding the use of both eye gaze tracking and speech tracking.

In operation 605, the diagnosis support system 100 may optionally determine whether a pre-detected patient parameter, such as a patient parameter detected by a sensor disposed in a waiting area, outside of the consultation room or even in the consultation room while the user was absent, is visible. For example, a patient parameter that presents on the fingers may not be visible if the patient's hands are in their pockets. As a further example, the patient may demonstrate a limp when they enter the consultation room. If the user (e.g. doctor) is not already in the consultation room when the patient enters, they may not be aware of the limp. In some embodiments, the sensor subsystem 120 may be configured to detect the presence of the user. If the patient parameter is not visible when the user is present, the diagnosis support system may determine that the user is not aware of the patient parameter even without the need to track the user's gaze. In this case, the diagnosis support system 100 may set the user awareness to unseen, as shown in operation 620. If the patient parameter is visible when the user is present, however, the method may proceed to operation 610.

In operation 610, the diagnosis support system 100 may determine whether the user's gaze is detected on an area or region of the patient's body corresponding to the detected patient parameter. If no gaze is detected on the area or region of the patient's body corresponding to the detected patient parameter, the user's awareness of the patient parameter is set to indicate that the patient parameter is unseen or unnoticed, as shown in operation 620.

If the user's gaze is detected on the area or region of the patient's body corresponding to the detected patient parameter, the diagnosis support system 100 may determine whether the gaze is detected for at least a first threshold time, as shown in operation 630. The first threshold time may be a predetermined number of seconds, for example. If the user's gaze is detected on said region or area for less than the first threshold, e.g. a shorter time than the first threshold time, then the user's awareness may be set to unseen or unnoticed, as shown in operation 620. The use of a first threshold time may be used to take into consideration accidental glances or skimming over a region.

If, on the other hand, the user's gaze is detected on said region or area for at least the first threshold time, the diagnosis support system 100 determines whether the user's gaze is detected on said region or area for more than a second threshold time, as shown in operation 640. The second threshold time is greater than the first threshold time. If the diagnosis support system 100 detects the user's gaze on said region or area for more than a second threshold time (such as a second predefined number of seconds defining a longer timespan than the first threshold time), then the user's awareness of the detected patient parameter may be set to indicate that the user has observed the patient parameter but is uncertain about it, as shown in operation 660. For example, if the user spends longer than the second threshold time looking at the corresponding area or region of the patient's body, the user may be unsure of how to interpret the patient parameter.

If the user's gaze is detected on the region or area of the patient's body corresponding to the detected patient parameter for some time greater than the first threshold time but less than the second threshold time, the diagnosis support system 100 may determine that the user has noticed the patient parameter with no uncertainty. This is illustrated in operation 650.

If, regardless of the duration of the user's gaze detected on the area or region of the patient's body corresponding to the detected patient parameter, the diagnosis support system 100 detects the user's gaze on said area or region at least a threshold number of times (shown in operation 670), then the system 100 may determine that the user is uncertain about the detected patient parameter, as shown in operation 660. For example, a user returning his or her gaze to the relevant area multiple times may be an indicator of uncertainty regarding the detected patient parameter.

In an embodiment, the user's voice may also be tracked, and the speech of a user may also contribute to the determination of the user's awareness. For example, if the user discusses the detected patient parameter, the diagnosis support system 100 may determine that the user is aware of the detected patient parameter. In another example, if the user discusses the detected patient parameter at length, for example asking the patient questions about the corresponding area or region of the body, then the diagnosis support system 100 may determine that the user is uncertain about the detected patient parameter, which may indicate that additional information is desired.

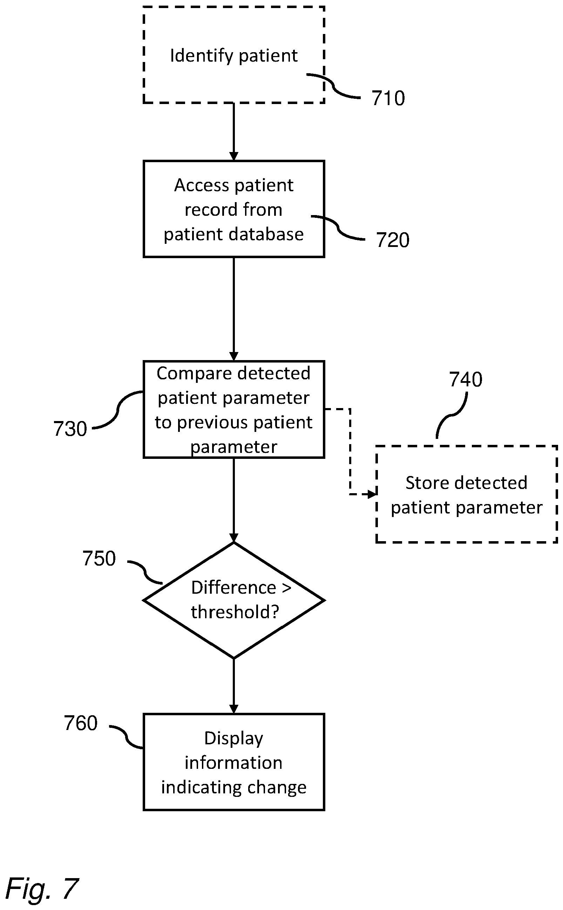

**Figure 7** schematically shows an example of an embodiment of a diagnosis support method. The method illustrated in Figure 7 may be used in conjunction with any or all of the steps of the methods of Figures 5 and 6.

In operation 710, the diagnosis support system 100 may be optionally configured to identify a patient. The patient identification may be performed using facial recognition via the sensor subsystem 120, and/or by referring to scheduling information. For example, if a user's (e.g. doctor's) schedule indicates an appointment time of 12:30 for John Smith, the patient seen by the user at 12:30 may be identified as John Smith. The timeliness of the user may additionally be tracked by the diagnosis support system 100 or an external device, by detecting the start and end times for each consultation.

The operation 710 of identifying the patient is optional. Alternatively, the user may merely input an identifier (e.g. name, social insurance number, etc.) to identify the patient. As a further example, biometric information such as fingerprints and/or iris scans may be used to identify the patient.

In operation 720, the diagnosis support system 100 may access a patient record corresponding to the patient, from a patient database. As discussed previously, the patient database may be stored in a memory of the diagnosis support system 100 or externally.

In operation 730, the diagnosis support system 100 may compare the detected patient parameter to a corresponding previous patient parameter. For example, if the detected patient parameter is a breathing rate, the diagnosis support system 100 may compare the patient's current breathing rate to a breathing rate previously stored in the patient's patient record. In an embodiment, the detected patient parameter may be stored in the patient record for future reference, as illustrated in operation 740.

In operation 750, the diagnosis support system 100 determines if the difference between the detected patient parameter and the corresponding previous patient parameter exceeds a threshold difference. The threshold difference may be set based on the type of parameter. For example, each parameter may have a predetermined range corresponding to an acceptable fluctuation. The threshold difference may be a set value or the threshold difference may be a percentage value. The threshold difference may be based on an accepted range for the patient parameter.

If the difference between the detected patient parameter and the corresponding previous patient parameter exceeds the threshold difference, the diagnosis support system 100 may be configured to display information indicating the change, in operation 760.

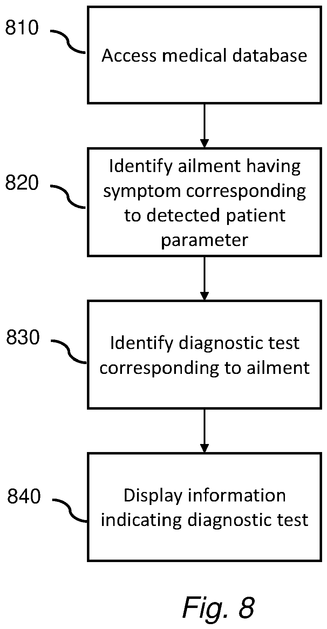

**Figure 8** schematically shows an example of an embodiment of a diagnosis support method.

The method of Figure 8 may be used in conjunction with any or all of the steps of the methods of Figures 5 through 7.

In operation 810, the diagnosis support system 100 may access a medical database, such as medical database 153 held in memory 150. The medical database may be a remotely maintained and/or hosted database, such as a central or governmental health database or the like.

In operation 820, the diagnosis support system 100 may identify at least one ailment or disease having a symptom corresponding to the detected patient parameter. In an embodiment, multiple patient parameters may be detected and the diagnosis support system 100 may identify at least one ailment or disease having some or all of the detected patient parameters.

In operation 830, the diagnosis support system 100 may identify a diagnostic test corresponding to the identified at least one ailment or disease. The diagnostic test may be used to obtain additional necessary information to assist the user in arriving at a diagnosis.

In operation 840, the diagnosis support system 100 may be configured to display information corresponding to the identified diagnostic test to the user. For example, the diagnosis support system 100 may prompt the user to ask further (e.g. specific) questions or to perform additional tests.

Many different ways of executing the methods shown in Figures 5, 6, 7 and 8 are possible, as will be apparent to a person skilled in the art. For example, the steps can be performed in the shown order, but the order of the steps may also be varied or some steps may be executed in parallel. Moreover, in between steps other method steps may be inserted. The inserted steps may represent refinements of the method such as described herein, or may be unrelated to the method. For example, steps 510, 520, 530 and 540 may be executed at least partially in parallel. Moreover, a given step may not have finished completely before a next step is started.

Embodiments of the method may be executed using software, which comprises instructions for causing a processor system to perform the methods of Figures 5 through 8. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

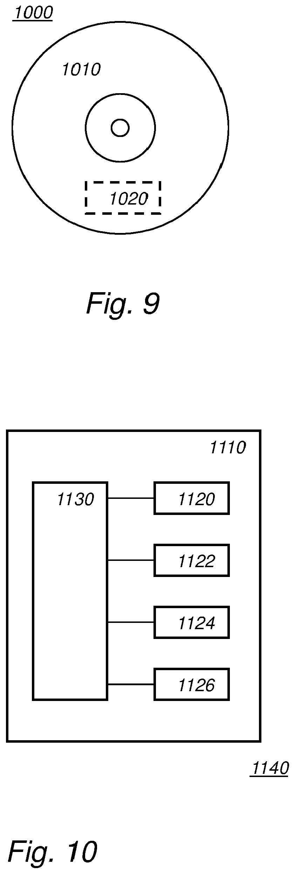

**Figure 9** shows a computer readable medium 1000 having a writable part 1010 comprising a computer program 1020, the computer program 1020 comprising instructions for causing a processor system to perform a method, such as any or all of the methods of Figures 5 through 8. The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said method of providing diagnosis support to a user.

**Figure 10** shows in a schematic representation of a processor system 1140, an example of processor subsystem 110, according to an embodiment of the diagnosis support system 100. The processor system comprises one or more integrated circuits 1110. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit (IC) 1124 for performing part or all of the processing defined in the method. Processor 1120, memory 1122, dedicated IC 1124 and communication element 1126 may be connected to each other via an interconnect 1130, for example a bus. The processor system 1110 may be arranged for contact and/or contact-less communication, using an antenna and/or connectors, respectively.

For example, in an embodiment, processor system 1140, e.g., the <claim 1> device may comprise a processor circuit and a memory circuit, the processor being arranged to execute software stored in the memory circuit. For example, the processor circuit may be an Intel Core i7 processor, ARM Cortex-R8, etc. In an embodiment, the processor circuit may be ARM Cortex M0. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

While device 1100 is shown as including one of each described component, the various components may be duplicated in various embodiments. For example, the processor 1120 may include multiple microprocessors that are configured to independently execute the methods described herein or are configured to perform steps or subroutines of the methods described herein such that the multiple processors cooperate to achieve the functionality described herein. Further, where the device 1100 is implemented in a cloud computing system, the various hardware components may belong to separate physical systems. For example, the processor 1120 may include a first processor in a first server and a second processor in a second server.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of' when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

## Claims

1. A diagnosis support system comprising:
- a sensor subsystem configured to detect a gaze of a user of the diagnosis support system and to monitor a patient;
- a display subsystem;
- a processor subsystem configured to:
- track, via the sensor subsystem, the gaze of the user;
- detect a patient parameter by monitoring the patient using the sensor subsystem;
- determine an awareness level for the detected patient parameter based on the tracked gaze, the awareness level indicating the awareness level of the user with regard to the patient parameter;
- if the awareness level of the patient parameter is below a threshold awareness:
- display, via the display subsystem, information indicating the detected patient parameter.

2. The system of claim 1, wherein the sensor subsystem further comprises a sound sensor configured to detect sound emitted by the patient, and wherein the patient parameter comprises at least one of: a speech pattern, a wheezing, a breathing, a heart sound and a coughing.

3. The system of claim 1 or claim 2, wherein the display subsystem comprises a head-mounted display (HMD).

4. The system of any preceding claim, wherein the patient parameter comprises at least one of: a gesture, a facial expression, a clothing characteristic, a hair characteristic, a fingernail characteristic, a temperature, a blood pressure, a tendon reflex, a heart rate, a breathing rate, a complexion, a weight, a gait and a physical anomaly such as a swelling or a discoloration.

5. The system of any preceding claim, wherein if the gaze of the user is detected on an area of the patient corresponding to the patient parameter for less than a first threshold time, the processor subsystem is configured to classify the awareness level of the patient parameter as unobserved.

6. The system of any preceding claim, wherein if the gaze of the user is detected on an area of the patient corresponding to the patient parameter for greater than a second threshold time, if the gaze of the user is detected on the area of the patient corresponding to the patient parameter at least a predetermined number of times, or if the patient parameter is not visible when the user is present, the processor subsystem is configured to classify the awareness level of the patient parameter as uncertain, and the processor system is further configured to display, via the display subsystem, information indicating said patient parameter.

7. The system of claim 5 or claim 6, wherein the processor subsystem is further configured to:
- collect gaze time information indicating a time required for the user to notice the patient parameter, and
- adjust at least one of the first threshold time and the second threshold time based on the collected gaze time information.

8. The system of any preceding claim, wherein the sensor subsystem is configured for speech recognition, and wherein the processor subsystem is configured to:
- recognize speech of the user using the speech recognition, and
- determine the awareness level of the detected patient parameter additionally based on the recognized speech.

9. The system of any preceding claim, wherein the system comprises a mobile device and wherein the display subsystem comprises a screen of the mobile device.

10. The system of claim 9, wherein the sensor subsystem comprises a camera of the mobile device, wherein the screen is arranged to show a live image of the patient, and wherein the information is displayed as an overlay on the live image of the patient.

11. The system of any preceding claim, further comprising a data interface configured to access a memory comprising a patient database, and wherein:
- the sensor subsystem is further configured for facial recognition; and
- the processor subsystem is further configured to:
- identify the patient using the facial recognition; and
- access a patient record corresponding to the identified patient from the patient database.

12. The system of claim 11, wherein:
- the processor subsystem is further configured to compare the patient parameter with a previous patient parameter stored in the patient record; and
- if the patient parameter differs from the previous patient parameter stored in the patient record by at least a threshold amount, display, via the display subsystem, information on the difference between the patient parameter and the previous patient parameter.

13. The system of claim 11 or claim 12, wherein the processor subsystem is further configured to store information of the patient parameter in the patient database.

14. The system of any preceding claim, wherein the processor subsystem is further configured to identify at least one potential ailment based on the patient parameter, and to display, via the display subsystem, information indicating a diagnostic test based on the at least one potential ailment and the patient parameter.

15. A computer-implemented method for providing diagnosis support to a user, the method comprising:
- tracking, via a sensor subsystem, a gaze of the user;
- detecting, via the sensor subsystem, a patient parameter by monitoring a patient;
- determining an awareness level for the detected patient parameter based on the tracked gaze of the user, the awareness level indicating the awareness level of the user with regard to the patient parameter;
- if the awareness level of the patient parameter is below a threshold awareness:
- displaying, via a display subsystem, information indicating the detected patient parameter.

16. A transitory or non-transitory computer-readable medium comprising data representing instructions which, when executed by a processor system, cause the processor system to perform the method according to claim 15.
